Europäisches Patentamt

European Patent Office

Office européen des brevets

(11) Publication number : **0 469 897 A2**

# (12) EUROPEAN PATENT APPLICATION

(21) Application number : 91307050.4

(22) Date of filing : 31.07.91

(51) Int. Cl.⁵ : **C12N 15/13,** C12P 21/08, C07K 15/00

(30) Priority : 31.07.90 US 560566

(43) Date of publication of application :
05.02.92 Bulletin 92/06

(84) Designated Contracting States :
AT BE CH DE DK ES FR GB GR IT LI LU NL SE

(71) Applicant : THE WISTAR INSTITUTE
36th Street at Spruce
Philadelphia Pennsylvania 19104 (US)

(72) Inventor : Rovera, Giovanni
933 Wootton Road
Bryn Mawr, Pennsylvania 19010 (US)
Inventor : Caton, Andrew J.
3706 Spring Garden Street
Philadelphia, Pennsylvania 19104 (US)
Inventor : Curtis, Peter J.
2401 Pennsylvania Avenue, Apartment 9C49
Philadelphia, Pennsylvania 19130 (US)
Inventor : Weiner, David B.
23 Henley Road
Pennwyne, Pennsylvania 19151 (US)

(74) Representative : Hartley, David et al
Withers & Rogers 4 Dyer's Buildings Holborn
London, EC1N 2JT (GB)

(54) Engineered antibodies.

(57) A DNA library comprising the variable region of immunoglobulin light chains and a DNA library comprising the variable region of immunoglobulin heavy chains are obtained. A prokaryotic cell is transformed with plasmid expression vectors containing one of said DNA libraries and the transformed cell is then infected with bacteriophage expression vectors containing the other of said DNA libraries. Infected and transformed cells express proteins. Proteins, including novel proteins, which bind to particular moieties can be selected by screening Fab proteins expressed by infected and transformed cells.

EP 0 469 897 A2

## BACKGROUND OF THE INVENTION

Field of the Invention

This invention is concerned with the expression and detection of recombinant high affinity binding proteins that are made up of two different immunoglobulin subunits. In particular, this invention concerns a method for selection of DNA sequences encoding immunoglobulin Fab proteins with affinity for particular moieties.

Summary of Related Art

It has recently been reported that, by use of appropriate translational and export signals in recombinant vectors, prokaryotes such as E. coli can be directed to translate and secrete correctly folded immunoglobulin (Ig) fragments. In Skerra, et al., Science, 240:1038-1041, (1988), DNA sequences encoding leader or signal peptides from genes such as ompA and phoA, were incorporated at the beginning of each of the separate sequences encoding the variable region of the heavy chain ($V_H$) and the variable region of the light chain ($V_L$) which made up the particular the $F_v$ fragment recognizing a specific antigen. These combined sequences were inserted into an expression vector which was subsequently used to transform E. coli cells. Culture filtrate recovered from the growth medium of the transformed cells contained $F_v$ fragments which were specific for the same antigen. In similar experiments, Better, et al., Ibid., 1041-1043, using $F_d$ and κ sequences with pelB leaders demonstrated that E. coli could secrete a properly folded Fab protein. This indicated that E. coli was capable not only of translating the amino acid sequence corresponding to the coding sequence for the immunoglobulin chains but also could transport the individual peptide sequences into the periplasmic space where the signal sequences were cleaved from the peptides and disulfide bonds were correctly formed before a properly folded $F_v$ or Fab fragment was ultimately secreted into the medium. The problem of producing in vitro a population of binding proteins whose diversity matches or exceeds the diversity inherent in the antibody population in vivo therefore reduces to the problem of expresing, in varying combinations, the heavy and light chains represented in the human lymphocyte genome.

When a pluripotent hematopoietic stem cell differentiates into cells of the B-cell lineage, the first detectable genetic event is an immunoglobulin heavy chain (IgH) gene rearrangement involving the variable, diversity, and joining regions of the heavy chain ($V_H$, $D_H$, and $J_H$, respectively), consisting of two successive somatic recombinations, $D_H$-$J_H$ and $V_H$-$D_H$-$J_H$ (Waldman (1987) Adv. Immunol, 40:247-321). A diverse and unique $V_H$ region is always generated during the formation of a complete $V_H$ gene. The large number of $V_H$, $D_H$, and $J_H$ segments can be randomly combined, and the flexibility arising at the recombination sites can include the insertion and deletion of heterogeneous, non-template-derived nucleotides. In addition, somatic mutation can further diversify the rearranged gene segments. Accordingly, an assembled $V_H$ region contains a framework of relatively conserved amino acid sequences that are interrupted by three hypervariable regions called complementarity-determining regions (CDR). Similar rearrangement generates the light chain variable region.

A large number of laboratories have attempted cloning of nucleic acid sequences corresponding to various immunoglobulin domains from both genomic DNA and mRNA sources since the mid-1970's. Cloning is complicated by the highly diverse nature of the immunoglobulin sequences. Nucleic acid amplification schemes such as the polymerase chain reaction (PCR) have been used to circumvent this problem. For example, short, highly conserved sequences flanking the highly diverse variable regions of the immunoglobulin genes have been used to generate oligonucleotide primers for the polymerase chain reaction. Using these primers it has been possible to amplify the nucleic acid sequences corresponding to fragments of immunoglobulins without reducing the heterogeneity of the somatic rearrangements of the genes coding for immunoglobulin. Orlandi, et al., Proc. Natl. Acad. Sci. USA, 86:3833-3837 (1989); Yamada, et al., Ibid., pp. 5123-5127; Sastry, et al., Ibid., pp. 5728-5732.

Ward, et al. (1989), Nature 341:544-546, incorporated a PCR-amplified population of different $V_H$ genes into plasmid expression vectors for E. coli, containing the appropriate secretory signal sequences. Cells transformed with these expression vectors secreted properly folded heavy chains into the medium and produced a wide range of different $V_H$ proteins. From a single plasmid library, Ward, et al., were able to select a number of different clones binding to each of two different antigens, indicating that this library contained sequences of substantial diversity, coding for $V_H$ proteins with a wide range of specific binding properties. It was suggested by Ward, et al. (and also by Austin, Nature, 341:484-485 (1989) in commenting on the results of Ward, et al.), that binding proteins of higher affinity could probably be produced by use of a similar approach in which E. coli clones of transformed cell, expressing both heavy, and light chain immunoglobulin domains, excreted Fab fragments formed from light and heavy chain proteins linked together by disulfide bridges in the manner described by Skerra, et al., and Better, et al.

Huse, et al. (1989), Science 246: 1275, taught one method of producing immunoglobulin Fab fragments containing heavy and light V regions and part of the constant region by constructing a bacteriophage DNA library in which a DNA sequence encoding one light chain was coupled to a DNA sequence encoding one heavy chain, where the light and heavy chain sequences from separate gene libraries of the sequences were combined randomly in vitro and then introduced into the bacteriophage by in vitro construction techniques. Subsequently, a susceptible host cell was infected with the resultant phage, and Fab fragments of diverse specificity were indeed produced. Huse et al., used libraries of DNA sequences encoding the light and heavy chain fragments obtained initially by PCR amplification of mRNA.

## SUMMARY OF THE INVENTION

It is an object of the present invention to provide an improved method for generating (and screening) a diverse population of cell expressing a population of Fab molecules in which a variety of heavy and light chains have been associated in random assortment to create novel binding proteins with a wide range of specificities.

In the method of this invention, an appropriate host cell is transformed with a plasmid expression vector population comprising a library of sequences encoding at least the variable region of either light or heavy immunoglobulin chains. The plasmid library-containing host cell population is infected with a bacteriophage expression vector population comprising a library of sequences encoding at least the variable region of the other of either light or heavy immunoglobulin chains. Fab molecules representing random combinations of the heavy and light chains in the two libraries can be recovered from plaques. The plaques are screened for Fab molecules with the desired specific affinity for a particular moiety, using any of the available methodologies described in the literature.

In a preferred mode, a library of sequences encoding the light chain is incorporated into a plasmid vector, and the library of sequences encoding the heavy chain is incorporated into a phage vector. Cells which are appropriate hosts for both vectors are transformed first with the plasmid vector. In a second step, cells which now contain the plasmid vector are then infected by the phage. Plates on which the infected cells are grown are screened for the presence of specific Fab molecules which bind with a particular moiety, as desired.

The method of this invention provides a rapid method for shuffling DNA sequences and selecting those which encode Fab molecules with desired binding activity. The library can be constructed from genomic DNA rather than from mRNA as was done by Huse, et al. Use of genomic DNA has an advantage because the mRNA population may be biased in favor of sequences coding for immunoglobulins with a limited set of specificities, depending on the prior immune exposure of the individual. However, the rearranged genomic DNA sequences are likely to contain broader diversity, reflective of a lymphocyte population which is unselected and not amplified.

By using different vectors for the heavy and light chain, and by sequentially introducing said vectors into a host cell, the method can be readily monitored at each step. Moreover, the method of this invention is much easier to accomplish than a method wherein the DNA for both the heavy chain and the light chain is incorporated into a single vector. This is because the construction of a single vector requires cumbersome, lengthy and difficult genetic manipulation in vitro, while a method using separate vectors can rely on cellular machinery to accomplish simultaneous expression.

## BRIEF DESCRIPTION OF THE FIGURES

Figure 1. The double stranded DNA sequence used to introduce the PelB leader peptide into expression vectors.

Figure 2A. A map of the vector mp19-Pel-$C_\gamma 1$ or -$C_\gamma 2$.

Figure 2B. A map of the vector pUC-Pel-$C_\kappa$ or -$C_\lambda$.

## DETAILED DESCRIPTION

This invention contemplates a rapid method for identifying novel binding proteins by screening a heterogeneous population of Fab molecules where the heavy and light Ig polypeptides that make up the Fab's are produced by random association of two heterogenous populations of DNA encoding the respective chains. The invention contemplates construction of highly heterogeneous libraries of DNA comprising the coding sequences for the variable region of individual immunoglobulin heavy chain and light chain polypeptides, preferably by amplification from genomic DNA. Light chain and heavy chain sequences are then inserted into two different prokaryotic expression vectors. These specialized vectors contain promoters as well as initiation, translation and export signal sequences at the 5′ end of the insertion point to direct secretion of the expressed polypeptides.

They preferably also contain the light chain constant region or a portion of the heavy chain constant region in translational reading frame on the 3′ side of the insertion point.

The invention contemplates the use of two separate vectors wherein one of vectors is a plasmid and the other is a phage and where the library corresponding to the Ig light chain is introduced into one vector and the library corresponding to the Ig heavy chain is introduced in the other vector. The present invention creates two separately microheterogeneous vector populations where the microheterogeneity results from the heterogeneity of the DNA libraries of the immunoglobulin gene variable regions. Cells of an appropriate host strain are first transformed with the plasmid vector containing one library and subsequently infected with the phage vector containing the other library. Expression of the Ig sequences of the vectors is induced, and light and heavy chain polypeptides encoded by the libraries are secreted as a population of Fab fragments capable, respectively, of specific binding to a diverse population of chemical moieties.

While fermentation of infected, transformed cells can be used as a source of these binding proteins, the primary use of this invention is as a screening system. Once transformed cells which secrete Fab proteins with the desired binding specificity and affinity are identified, the desired DNA sequences contained in these cells can then be introduced into more efficient production systems using well-known recombinant techniques (e.g., Winter, Eur. Pat. Appl. 0 239 400, published Sept. 30, 1987; Cabilly, et al., Eur. Pat. Appl. 0 125 023, published Nov. 14, 1984).

A. Generation of Ig gene libraries

Two separate gene libraries, corresponding to the rearranged variable regions of the Ig heavy chain and the Ig light chain genes, respectively, are prepared. The rearranged immunoglobulin DNA variable regions of genomic DNA may be amplified by PCR to produce variable region cassettes which contain sequences encoding all of the variable regions in the rearranged genome. Other methods for amplifying DNA corresponding to the immunoglobulin variable regions may also be used (see, e.g., Van Brunt, Bio/Technol., 8:291-294, 1990; Guatelli, et al., Proc. Natl. Acad. Sci. USA, 87:1874-1878, 1990). In a less preferred alternative, the corresponding variable regions from lymphocyte mRNA can be amplified by any appropriate nucleic acid amplification method.

When amplifying by PCR, conserved regions at each end of the nucleotide sequences which encode variable domains (V) of immunoglobulin heavy chain ($V_H$) and light chain ($V_L$) are first identified. Second, primers for the amplification, which incorporate restriction sites for forced cloning, are designed. Third, vectors containing these same restriction sites are constructed that allow the amplified DNA to be replicated and expressed, while retaining the amino acid sequence typical of V domains.

The $V_H$ segments in the human $V_H$ locus, comprising about 100-200 members, have so far been grouped into six families ($V_H$ 1-6). Members of each family are highly interspersed, with a distance of at least 3.5-11 kilobases (kb) between two adjacent $V_H$ segments (Berman, et al., (1988) EMBO J., 7:727-738). The $V_H$ locus is 2500 kb in size (Berman, et al., 1988) and is separated from the $J_H$ locus by <100 kb. The $J_H$ locus, 3 kb in size, consists of a cluster of six segments and is 6 kb upstream of the constant region of the μ-chain gene (Ravetch, et al., (1981) Cell, 27:583-591). The $D_H$ regions can be classified into six different $D_H$ families, with great divergence of nucleotide sequence among different family members. The six $D_H$ genes are multiplied at least five times and are dispersed among $V_H$ clusters (Ichihara, et al., (1988) EMBO J., 7:4141-4150). When a pluripotent hematopoietic stem cell differentiates into cells of the B-cell lineage, the first detectable genetic event is an immunoglobulin heavy chain (IgH) gene rearrangement involving the variable, diversity, and joining regions of the heavy chain ($V_H$, $D_H$, and $J_H$, respectively), consisting of two successive somatic recombinations, $D_H$-$J_H$ and $V_H$-$D_H$-$J_H$ (Waldman (1987) Adv. Immunol, 40:247-321). These changes bring a $V_H$ segment next to a $J_H$ segment at a distance of <1000 bases. Similar rearrangements generate the $V_L$ region during stem cell differentiation.

The polymerase chain reaction (PCR) (Saiki, et al., (1985) Science, 230:1350-1354) has been used recently for genomic (Scharf, et al. (1986) Science, 233:1076-1078) and cDNA cloning (Saiki, et al., (1988) Science, 239:487-491). It involves repeated rounds of extension from two primers specific for regions at each end of the gene. The primers need not match the gene sequence exactly (Lee, et al., (1988) Science, 239:1288-1291), and restriction sites can be incorporated within the primers to allow the forced cloning of the amplified DNA (Scharf, et al. (1986) Science, 233:1076-1078).

The preferred approach of the invention uses the PCR technology to amplify the rearranged regions of the variable immunoglobulin genes, without introns, and uses custom-made oligonucleotide primers to produce V region cassettes corresponding to the entire coding region of the respective heavy and light chain variable regions. Amplification of the rearranged variable regions at the genomic level is feasible by using oligonucleotides that have homology to the conserved sequences in the FR1 region of $V_H$ gene segments and the 3′ region of

$J_H$ segments. Accordingly, the inability of the PCR to operate over ranges greater than 3-6 kb (Saiki, et al., (1985) Science;, 230:1350-1354; Saiki, et al., (1988) Science, 239:487-491) results in these oligonucleotides directing only the amplification of the rearranged regions because these sequences are widely separated in stem cell genomic DNA and come into close proximity only after $V_H$-$D_H$-$J_H$ rearrangement. Quantitation of the positive recombinant clones has been shown to accurately reflect the number of given CDR-III DNA sequences and, therefore, of specific B-cell clones in the whole population (Yamada, et al., 1989).

V gene oligonucleotide primers are designed based on sequences of framework region 1 (FR1) which display sequence homology with members of the $V_H$ and $V_L$ families thus far identified. The $J_H$ and $J_L$ antisense primers are designed to match the 3′ ends of the $J_H$ or $J_L$ segments. Sequences for the primers can be selected from the conserved portion of the variable region sequences given in Kabat, et al. (1987) Sequences of Proteins of Immunological Interest, U.S. Dept. of Health and Human Services, Government Printing Office as described by, e.g., Sastry, et al.

The 5′ oligonucleotide primer sequences should contain enough of the 5′ sequence to ensure hybridization to the sequence encoding the first amino acid of each published sequence. Similarly, 3′ oligonucleotide primer sequences are designed to hybridize to the 3′ end of all known J sequences in the species of interest. While these 5′ and 3′ regions are highly conserved, so that relatively few distinct sequences occur in these regions, multiple primers will be necessary, especially on the 5′ end, to provide sequences which hybridize to all or even a plurality of the distinct V and J segments. The PCR reagent mixture should optimally contain multiple primers to ensure capturing, within the V region capsule, the diversity inherent in the rearranged lymphocyte genome. Alternatively, separate reactions can be carried out using different combinations of primers.

Primers are modified by introducing point mutations, preferably near their 5′ end (Scharf, et al., 1986), to generate restriction sites. The presence of artificial restriction sites in the primers will allow the cloning of the V capsules into plasmid or phage vectors. The restriction sites incorporated into the primers correspond to restriction sites in the vectors that will be used to clone and express each V capsule library. Unique sites may be present in the respective heavy and light chain vectors, to permit independent manipulation of each library.

The unique 5′ and 3′ primer sequences should preferably be further multiplied (tripled) by including either 0, 1 or 2 additional nucleotides on the 3′ side of the restriction site to generate in-frame translation products once the variable region cassettes are ligated to the leader sequences and to the constant region sequences. The use of such primers will rescue additional diversity in the V regions of the genome that would otherwise be lost as a result of some rearrangement during stem cell differentiation that produced a shift of reading frame in the sequences following the rearrangement site. An alternative, less preferred method to accomplish the same rescue of diversity would be to insert 0, 1, or 2 added nucleotides in the vector on the 3′ side of the restriction site, next to the constant region. This alternate procedure may necessitate construction of three different vectors into which the library would be inserted.

## B. Construction of vectors

Two different expression vectors are used to introduce intronless DNA from the two libraries into the desired host cell: a plasmid vector and a bacteriophage vector. The library of sequences containing the amplified light chain variable region is inserted into one expression vector, and the library of sequences containing the amplified heavy chain variable region is inserted into the other expression vector. The population of host cells transformed and infected with the expression vectors containing the immunoglobulin variable region libraries produces a highly diverse population of Fab molecules. In a preferred made, the light chain variable region is inserted into the plasmid vector and the heavy chain variable region is inserted into the phage vector.

Vectors used in this invention are constructed from prokaryotic expression vectors which are known to transform or infect a host cell selected as described below and which have a well-characterized polycloning site. The plasmid and phage vectors preferably have limited homology to avoid exchange of genetic material. The vectors should preferably contain different markers to permit separate or simultaneous selection.

The vectors are each constructed to have, 3′ to the promoter that will be used to direct expression, a ribosome binding site followed by a secretory signal sequence that is effective in the host strain chosen for expression. In one preferred mode, the Pel B leader sequence which was used by Better, et al., can be used with E. coli host cells. Restriction sites, corresponding to the sites present in the PCR primers used to generate the $V_L$ or $V_H$ libraries, follow after the signal sequence such that the V region capsule will be inserted in translational frame. Immediately following these restriction sites, a sequence which encodes the constant region for the appropriate heavy or light chain and ends with a stop codon is preferably inserted. An appropriate constant region sequence can be readily selected from published sequences by the skilled worker. The nucleotide sequence corresponding to this sequence can be prepared, for example, by oligonucleotide synthesis or by PCR using primers designed from the published immunoglobulin sequences. Optionally an additional sequence can be added between the end of the constant region and the stop codon. This sequence will result in expression of an additional peptide tag attached to the expressed immunoglobulin peptide which may be used for identification or for screening.

By preparing the vectors with the constant region in place, variable region cassettes can be prepared from lymphocyte DNA, and after insertion into said vectors, a complete sequence for the light or heavy chain of the Fab fragment can be transcribed and translated in a prokaryotic system without need for removing intron sequences. In a less preferred alternative, variable region or complete chain sequences can be obtained from lymphocyte mRNA as reported by Huse, et al. However, this population is likely to be skewed in favor of antibodies specific for antigens to which the organism is sensitized, and therefore the ultimate diversity of the resulting Fab population will be reduced compared to the preferred approach.

It is not necessary that the promoters used be very active ones, because the purpose is screening for DNA sources, not high volume protein production. More efficient production systems can be constructed later, once the desired DNA sequences have been selected. The promoters used on the two vectors may be the same (for coordinate induction by a single chemical) or different (to permit individualized control of expression).

A wide variety of plasmids and phages are known in the art (e.g., Pouwels, et al., "Cloning Vectors," Elsevier, 1985). The choice of a particular plasmid and phage to be used in conjunction with any given host is easily within the skill of the art. Similarly, the choice of a promoter, a secretory signal sequence, location and type of restriction sites and stop codon are within the skill of the routineer.

A preferred plasmid vector for an E. coli host system is pUC19. A preferred phage vector is based on M13. The pUC19 plasmid vector carries an ampicillin resistance marker and has a multiple site inserted in the lac operon as does M-13, both available from e.g., American Type Culture Collection (ATCC) or Bethesda Research Laboratories.

## C. Transformation of host cells with plasmid vector

Host cells compatible with each of the vectors should be used for transformation. The preferred system is based on E. coli and E. coli cloning vehicles. The host cell's genetic makeup is preferably chosen to simplify selection procedures during cloning. The selection procedures may include, e.g., antibiotic resistance, or other known procedures. Vectors and host cell systems having the desired properties are described in Pouwel, et al. (1985) and readily available from a number of sources, e.g., ATCC, and the selection procedures are described, e.g., in Sambrook, et al., Molecular Cloning: A Laboratory Manual, 2nd ed., Cold Spring Harbor Laboratory Press, 1989, which is incorporated herein by reference.

The host cell is transformed with the recombinant plasmid carrying a library of immunoglobulin sequences, coding for one of the chains. The successful transformants are selected using standard techniques, preferably antibiotic resistance. Transformation, and selection of transformed cells, is within the skill of the ordinary worker and well described in the published literature, e.g., Sambrook, et al.

## D. Infection of transformed host cells

Infection of a population of transformed host cells carrying an expression library for one immunoglobulin polypeptide by a bacteriophage vector which includes an expression library for the corresponding immunoglobulin polypeptide can be accomplished by any one of a number of well-known methods which are used in the art for expression of a recombinant nucleotide sequence carried on a bacteriophage. In one method, a virus stock of the bacteriophage vector which includes the library for the other immunoglobulin chain is prepared, and a suspension of the host cells is mixed with the virus stock. The mixture of cells and bacteriophage is incubated to allow attachment of the virus particles to the cells, and then the cells are plated out. The plating medium may contain inducers for the expression system(s) of the two vectors selected. Alternatively, the plate may be overlaid with a filter pre-soaked in inducers for the promoters present on the two expression vectors.

In an alternative approach, transformed host cells carrying an expression library for one immunoglobulin polypeptide are plated out in lawns and then infected by the bacteriophage which includes an expression library for the immunoglobulin polypeptide corresponding to the other immunoglobulin chain. The infected lawn is then overlaid with nitrocellulose filter pre-soaked in inducers for the promoters present on the two expression vectors. In either method, the concentration of bacteriophage used will be adjusted to generate an appropriate number of plaques on the final plate, preferably about 20,000 per 15 cm plate.

After further incubation, plaques are screened for the presence of binding proteins specific for the particular moiety desired, using standard procedures. The particular moieties to which the Fab molecules generated during the screening phase bind include, but are not limited to antigens, haptens, epitopes and similar chemical structures capable of molecular recognition. One procedure described by Huse, et al. (1989) involved lifting the binding protein on nitrocellulose filters and performing binding assays on the filters, using iodinated antigen proteins as the moiety to which the Fab bound. For example, an inducer-soaked filter can be lifted from the colonies, blocked with irrelevant protein and then probed with labeled (either radiolabeled or enzymatically labeled) antigen. The specific detection events are then used to locate the DNA clones of interest from the master plate. The relevant plaques can then be picked and replated and rescreened to enrich for the relevant Fab producing colonies.

Alternatively, the filter paper could be coated with molecules containing the relevant moiety and blocked prior to incubation onto the lawn of infected bacterial cell. The recombinant Fab fragments bind to specific regions of the filter paper correlated with their colonies of origin and are then detected by antisera reactive with anti-FAB antibodies or with an encoded tag region included in the construct.

Once the plasmid-containing host cells and the bacteriophage which both contain immunoglobulin variable region libraries are available, the procedure for infecting the cells and screening expressed Fab fragments is so simple, the ordinary worker may prefer to simply perform a new bacteriophage infection experiment followed by screening each time a protein specific for a new moiety is required. Alternatively, after an initial screening for a first binding protein, Fab fragments, or molecules, which bind to additional moieties may be identified by testing the plaques for binding ability toward these other moieties in a subsequent step. In another alternative, different molecules containing other moieties for which binding proteins of interest are desired may be labelled with different, uniquely detectable tags that can be labelled to simultaneously detect in distinct plaques different Fabs binding to different moieties.

E. Recovery of DNA from transfected cell line

The bacteriophage and plasmids of the plaque identified with the binding protein of interest are recovered by standard methods. Identified bacteriophage can be recovered from plaques by standard methods for the isolation and plaque purification of bacteriophage. Plasmids can be recovered from plaques by, for example, publication of supercoiled DNA followed by transformation of new host bacteria and subsequent expansion and growth of independent colonies. Since each plaque can contain different bacterial cell that possess different plasmids, several different plasmid types may be independently isolated by growth of independent colonies. Colonies that contain each plasmid type can be expanded and again infected with the bacteriophage that was derived from the same plaque. Each bacteriophage/plasmid combination can be tested for its ability to bind the antigen, in order that the most desired plasmid/ bacteriophage combination can be identified.

In those cases where the primary determinants of binding specificity lie in the $V_H$ region, and the contribution of the $V_L$ region is to provide enhancement to the binding affinity of the Fab fragment, the method of this invention may be optimally practiced by placing the $V_H$ library in the bacteriophage vector and infecting a lawn of transformed bacteria. A plaque will then contain a variety of Fab fragments composed of a single heavy chain and a variety of light chains, increasing the chances that a heavy chain specific for the desired target will combine with the light chain that most enhances its affinity. Further, this may increase the probability of detecting the heavy chain, since more than one of the light chains in the region of the plaque may combine to produce Fab's of enhanced affinity, resulting in a more easily detected population of Fab's with the desired specificity.

Where the bacteriophage comprises the preferred M-13-based vector, transformed and infected cells are not lysed, so the cell which produces the Fab molecule with the highest affinity may be colonized by plating out cells of the plaque identified in the original binding study. The desired DNA sequences can then be recovered from the plasmid and bacteriophage present in the cells of the single cell colony which shows the highest binding affinity.

Appropriate bacteriophage and plasmid combinations can be identified and grown to large quantities. DNA can be isolated and the sequences encoding the heavy and light chain variable domains can be excised using the same restriction sites that were used in their initial introduction into the screening vectors. These sequences can then be inserted into any expression system; where necessary, they can be further modified prior to introduction into other expression systems through standard methods of mutagenesis to insert and/or remove restriction sites and/or other sequences as may prove effective in achieving maximal expression in any particular system.

Example 1 Preparation of Specialized Expression Vectors.

1) Insertion of Pel B in the Hind III - Sal I Cloning sites of pUC 19 and M13 mp 19.

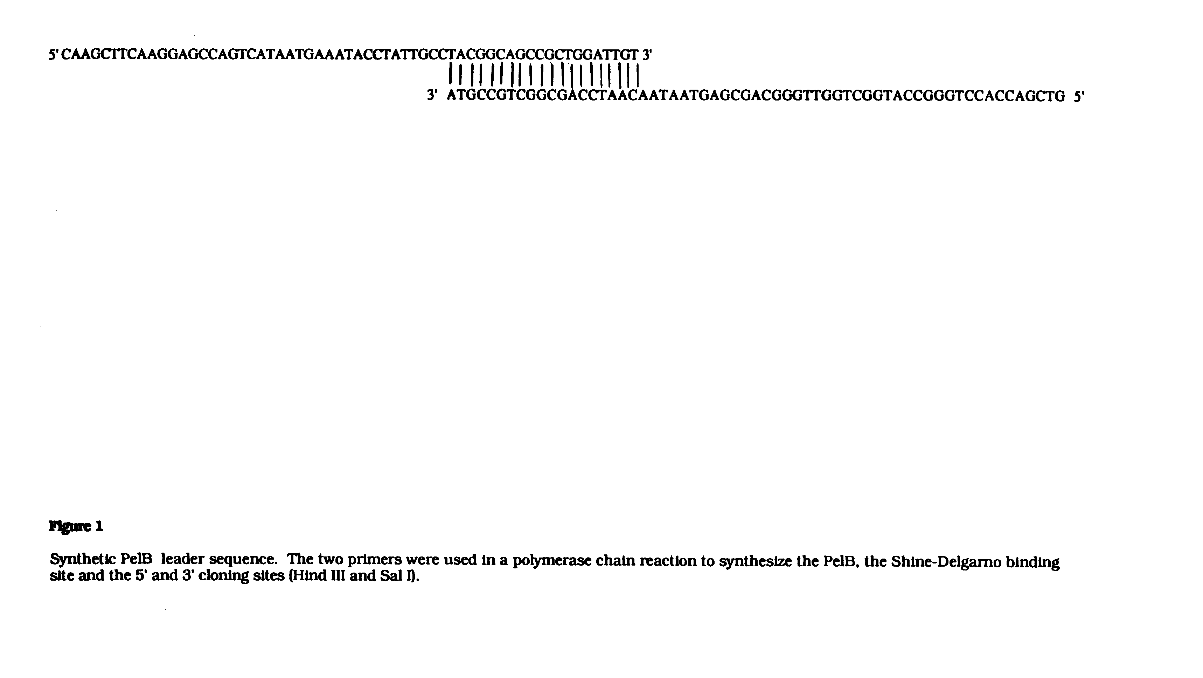

A synthetic PelB DNA containing at its 5' end a consensus Shine Delgarno sequence for efficient attachment of bacterial ribosomes and efficient translation, has been prepared by PCR amplification of 2 60-mer oligonucleotiedes as shown in Figure 1. The synthetic PelB has been force ligated into the Hind III - Sal cloning sites of the polylinker present in pUC 19 and M13 mp 19 vectors and transfected Into E. coli DH5 α F'IQ cells using established methods.

The two vectors obtained have been called pUC-PelB and mp19-PelB.

## 2) Synthesis of constant regions for κ and λ and Subcloning into the Kpn 1/EcoRI sites.

Using the primers Shownn in Table 3, the κ and λ constant regions have been amplified from human peripheral blood lymphocyte DNA using the polymerase chain reaction. The amplified material was ligated into the pUC-PelB vector by force cloning into the Kpn 1 and EcoRI sites. The vectors obtained have been called pUC-PelB-$C_\kappa$ and pUC-PelB-Cλ.

## 3) Synthesis of $C_H1$-$_\gamma$ and $C_H1$-$_\gamma$2 Chains and Cloning Into the KpnI and EcoRI Cloning Sites of mp19-PelB Vector

The segment of the constant region of the immunoglobulin heavy chain that codes for the sequence of constant region present in the Fab fragment is present in the first exon of the constant region gene. This segment (CH1), with adequately modified cloning sites, has been amplified using the primers shown in Table 4 and using as a template the DNA of peripheral blood human lymphocytes. The amplified material was force cloned in the mp19-PelB vector at the KpnI and EcoRI cloning sites.

After transformation in E. coli, the recombinant colonies were screened using diagnostic oligonucleotide probes specific for the $CH_1$-$_\lambda$1 and $CH_1$-$_\gamma$2 sequences (Table 4).

The identified positive clones were sequenced to confirm that the $CH_1$-$_\gamma$1 and $CH_1$-$_\gamma$2 sequences were correctly inserted into the vector.

The vector has been called mp 19-PelC-$_\gamma$2.

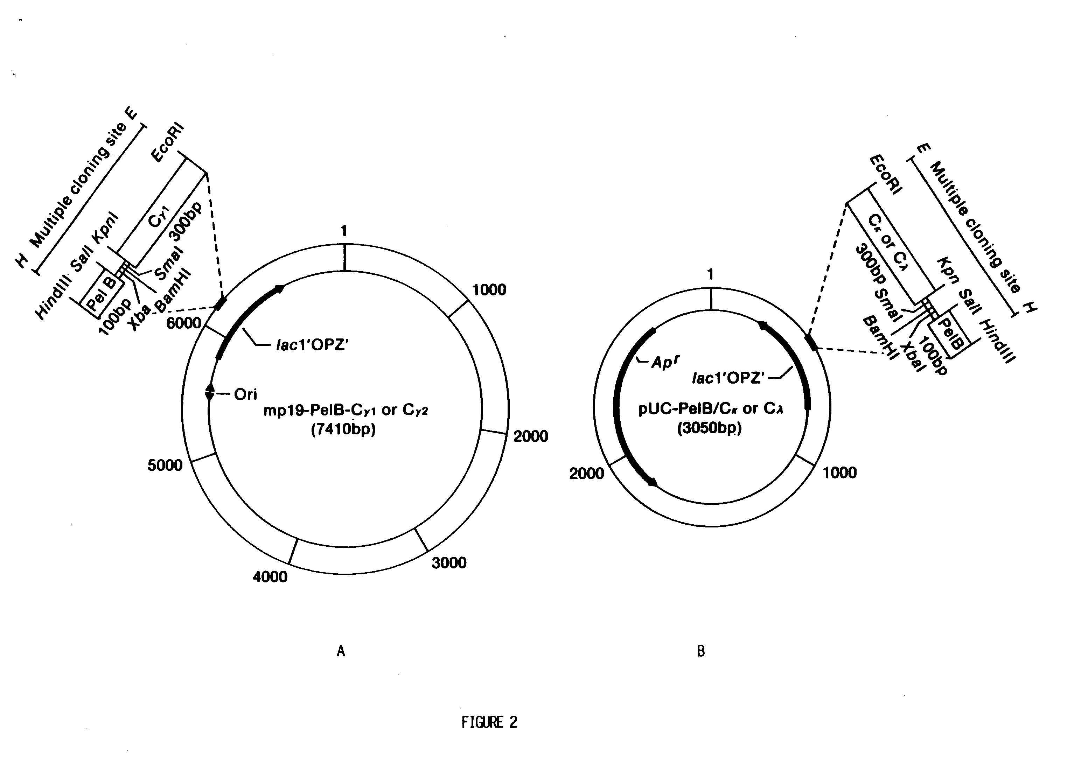

The maps of the four specialized vectors described here are Shown in Figure 2.

## Example 2 Preparation and Screening of Immunoglobulin Gene Libraries

## A) Preparation Libraries of Immunoglobin Sequences

## 1) Preparation of $V_H$-D-$J_H$ Joinings Pool - DNA

High-molecular-weight genomic DNA is isolated from spleen cells or peripheral blood lymphocytes by established procedures (Sambrook, et al. 1989). Genomic DNA sequences are amplified by the polymerase chain reaction according to Yamada et al. 1989.

PCR is carried out as described by Saiki et al. (1985, 1986). The DNA is initially denatured in boiling water for 5 minutes followed by addition of 5 units of Thermus aquatics (Taq) DNA polymerase (Perkin-Elmer/Cetus). Each cycle contains an annealing step at 55°C for 1 minute, an elongation step at 70°C for 2 minutes, and a denaturation step at 95°C for 2 minutes. A total of 30-40 cycles are carried out. Primers used for amplification of $V_H$-D-$J_H$ cassettes are listed in Table 1.

The amplified DNA is precipitated in 2 volumes of 100% ethanol and resuspended in buffer containing 10 mM Tris with 1 mM EDTA (pH 7.5), and one-fifth of the reaction product is analyzed by gel electrophoresis in 4% agarose gel (NuSieve, FMC) in 0.089M Tris borate/0.002 M EDTA. For Southern blotting the electrophoresed DNA is transferred to nylon membranes (Zetabind, Cuno) using 1 M ammonium acetate buffer (pH 8.0) and hybridized at 50°C to $^{32}$P-labeled diagnostic oligonucleotide probes which are homologous to the 5' end of J regions. After washing filters in 6X SSC (1X SSC = 0.15 M NaCl/0.015 M sodium citrate, pH 7.0) at 2°C below the calculated melting point of the oligonucleotide probes (Whitehead, et al., (1983) Proc. Natl. Acad. Sci. USA, 80: 5387-5391), filters are exposed to X-ray film at -70 °C overnight.

Impurities in the amplified mateiral are removed by Geneclean (Bio 101, La Jolla, CA) treatment. DNA is digested with Sal I and Kpn 1 and ligated to the specialized phage vector mp 19-PelC$_\gamma$-1 or mp19 Pe1C$_\gamma$-2 described below to generate a $V_H$ chain bacterophage library.

## 2) Preparation of $V_L$-$J_L$ Pool DNA.

A library of light chain sequences is prepared similarly except that a different set of primers are used in the polymerase chain reaction. The primers to be used for amplification of $V_L$-$J_L$ regions are shown in Table 2.

The amplified material is cloned into the specialized plasmid vector pUC-PelB-Cκ or pUC-PelB-$C_\lambda$ to gen-

erate a $V_L$ plasmid library.

B) Transfection of Host Cells With pUC PelB-$c_\kappa$ ($_\gamma$)

The host cell used for this example is obtained from Bethesda Research Laboratory and is designated DH5 $\alpha$ F[1] I Q. This is a male E. coli strain containing a stable $F_1$ episome and producing a-lac repressor protein which regulates transcription from the lac I promoter. These cells are transformed with specialized plasmid vectors pUC-PelB-$C_\kappa$ or pUC-PelB-$c_\lambda$ which contain the light chain library. Transformed cells are selected by growth in an ampicillin-containing medium.

C) Infection of the Transformed Host Cell With Bacteriophage Containing the Heavy Chain Library

A suspension of the selected transformed cells described in B above is mixed with a suspension of the specialized bacteriophage mp19 PelC-$_\gamma$ vectors containing the immunoglobulin variable heavy chain library prepared as described above and plated as described in Sambrook, et al. (1980). After incubation, plaques are lifted with nitrocellulose filters and screened for binding of the desired protein antigen, which has been labelled with $^{125}$I, using autoradiography as described by Huse, et al. (1989).

D) Screening and Recovery of DNA For Selected Fab

The section of agar in the plate corresponding to positive binding locations on the nitrocellulose filter are excised, DNA is extracted from them, and the resultant DNA extracts are amplified by PCR as described above.

Example 3 Alternate Screening Procedure

Transformed cells and bacteriophage containing the heavy chain library are prepared as described for Example 2 A.

Recombinant E. coli transfected with pUC-PelB-$C_\lambda$ specialized vector are plated out in lawns and then infected by the recombinant variable heavy chain-containing mp19-PelC-$_\gamma$ preparation. The infected lawns are then allowed to incubate until the average plaque density per plate is 20,000 single plaques per plate. The lawn is then overlaid with marked (for later orientation) nitrocellulose-based filters presoaked in IPTG for induction of the construct insert via the $\beta$-gal promoter. This initiates production of the secreted forms of the recombinant Fab's within the infected lawns. The IPTG soaked filter is lifted from the colonies, blocked with irrelevant protein and then probed with labeled (either radiolabeled or enzymatically labeled) antigen. The specific detection events are then used to locate the DNA clones of interest from the master plate. The relevant colonies can then be picked and replated and rescreened to correctly enrich for the relevant Fab producing colonies. It is clear that there can be several variations of this approach; for example, it is entirely possible to pick the relevant clones and PCR the inserted variable region cassettes directly. These variable regions can then easily be transferred to other expression systems for further studies and insert verification.

Example 4 Alternate Screening Procedure

The procedure is the same as Example 2, except that the nitrocellulose-based induction paper is coated with the relevant antigen and blocked prior to incubation on the lawn of infected bacterial cells. The recombinant Fab fragments bind to specific regions of the filter paper corresponding to their colonies of origin, which are then detected by antisera reacting with the encoded tag region of the construct or with anti-Fab antibodies. The identified colonies are then picked and rescreened to isolate relevant clones of interest.

Since modification of the invention will be apparent to one skilled in the art, it is intended that the invention be limited only by the scope of the appended claims.

## TABLE 1

### Primers for PCR amplification of $V_H$-D-$J_H$ joinings

$V_H$ 1-5 sense      T GTC GAC CAG GTG CAG CTG GTG C

$V_H$ 3 sense       T GTC GAC CAG TGT GAG CTG GTG GAG

$V_H$ 4-6 sense      T GTC GAC CAG GTG CAG CTG CAG GAG

$J_H$ antisense      T GGT ACC TGA GGA GAC GGT GAC C

Underlined are the Sal I cloning site (GTC GAC) and the Kpn I cloning site (GGT ACC).

## TABLE 2

### Primers for PCR amplification of $V_L$-$J_L$ joinings

$J_K$ antisense      T GGT ACC ACG TTT GAT TTC CAC CTT GGT CCC

$J\lambda$ antisense      T GGT ACC ACT TAG GAC GGT CAG CTT GGT CCC

$V_K$-1 sense      T GTC GAC GAC ATC CAG ATG ACC CAG TCT

$V_K$-2 sense      T GTC GAC GCT CAA GTG CTG ACC CAG ACT

$V_K$-3 sense      T GTC GAC GAT GTT GTG ATG ACC CAA ACT

$V_L$-1 sense      T GTC GAC CAG TCT GTG TTG ACG CAG CCG CCC

$V_L$-2 sense      T GTC GAC CAG ACT GTG GTG ACT CAG GAG CCC

Underlined are the Sal I (GTC GAC) and the Kpn I (GGT ACC) cloning sites.

TABLE 3

Primers for PCR amplification of $C_K$ and $C\lambda$ segments

$C_K$ sense          T <u>GGT ACC</u> GTG GCT GCA CCA TCT GTC

$C_K$ antisense      T <u>GAA TTC</u> **TTA TTA** ACA CTC TCC GTT GAA

$C\lambda$ sense     T <u>GGT ACC</u> CAG CCC AAG GCT GCC

$C\lambda$ antisense T <u>GAA TTC</u> **TTA TTA** TGA ACA TTC TGC AGG


Bolded are the bacterial stop codon sequences
Underlined are the KpnI (GGT AAC) and the EcoRI (GAA TTC) cloning sites.


TABLE 4


Primers for synthesis of $C_H$ 1-γ1 and $C_H$ 1-γ2 chains

CH 1-γ  sense       T <u>GGT ACC</u> TGA GGA GAC GGT GAC C

$C_H$ 1-γ antisense T <u>GAA TTC</u> **TTA TTA** TTT CTT GTC CAC CTT GGT

C1 DP               ACC TCT GGG GGC ACA GCG GC

C2 DP               ACC TCC GAG AGC ACA GCC GC


Bolded are the prokaryotic stop codons.
Underlined are the EcoRI (GAA TTC) and the KpnI (GGT ACC) cloning sites.


**Claims**

1.  A method for selecting DNA sequences which encode proteins containing at least two different subunits and which are capable of binding to a particular moiety comprising the steps of:
    a) transforming a prokaryotic cell with a population of plasmid expression vectors containing a first DNA library;
    b) infecting said transformed cell with a population of bacteriophage expression vectors containing a second DNA library;
        wherein one of said DNA libraries comprises the variable region of immunoglobulin light chains and the other of said DNA libraries comprises the variable region of immunoglobulin heavy chain; and
    c) identifying infected, transformed cell which express proteins that bind to said moiety.

2.  The method of claim 1 wherein the infected, transformed cell are incubated to express proteins comprising at least one subunit encoded by DNA contained in one of the two DNA libraries and at least one subunit encoded by DNA contained in the other of the two DNA libraries, where the subunits have been associated in random assortment.

3.  The method of claim 1 wherein the plasmid contains the DNA library for immunoglobulin light chain and the bacteriophage contains the DNA library for immunoglobulin heavy chain.

4. The method of claim 1 wherein said DNA library comprising the variable region of the immunoglobulin light chain is obtained by amplifying the rearranged variable region in DNA isolated from lymphocytes.

5. The method of claim 1 wherein said DNA library comprising the variable region of the immunoglobulin heavy chain is obtained by amplifying the rearranged variable region in DNA isolated from lymphocytes.

6. The method of claim 1 wherein both said expression vectors also contain DNA sequences encoding an immunoglobulin constant region, where the light chain library is in translational frame with light chain constant sequences and the heavy chain library is in translational frame with heavy chain constant sequences.

7. The method of claim 1 wherein step (c) further comprises the steps of:
   a) growing the infected, transformed cell under conditions which induce expression of the proteins encoded by said expression vectors;
   b) determining which cell express proteins which bind to said moiety;
   c) recovering DNA from cells identified in step b.

8. The cell identified in the method of claim 1.

5' CAAGCTTCAAGGAGCCAGTCATAATGAAATACCTATTGCCTACGGCAGCCGCTGGATTGT 3'
|||||||||||||||||||||||||||
3' ATGCCGTCGGCGACCTAACAATAATGAGCGACGGGTTGGTCGGTACCGGGTCCACCAGCTG 5'

EP 0 469 897 A2

**Figure 1**

Synthetic PelB leader sequence. The two primers were used in a polymerase chain reaction to synthesize the PelB, the Shine-Delgarno binding site and the 5' and 3' cloning sites (Hind III and Sal I).

FIGURE 2

EP 0 469 897 A2